# EUROPÄISCHE PATENTSCHRIFT

(11) **EP 3 846 929 B1**
(45) Veröffentlichungstag und Bekanntmachung des Hinweises auf die Patenterteilung: **23.11.2022**
(21) Anmeldenummer: 19762156.8
(22) Anmeldetag: 03.09.2019
(51) Int. Cl.: B01J 10/00, B01J 19/18, B01J 19/26, B01J 19/30, C07C 29/17, C07C 29/56, C07C 45/62, C07B 53/00, B01J 19/24, B01J 19/32, B01J 19/00, B01J 3/04

(54) **REAKTOR ZUR DURCHFÜHRUNG EINER GAS/FLÜSSIG-ZWEIPHASIGEN HOCHDRUCKREAKTION MIT EINEM SCHÄUMENDEN MEDIUM**
REACTOR FOR CARRYING OUT A GAS-LIQUID TWO-PHASE HIGH-PRESSURE REACTION WITH A FOAMING MEDIUM
RÉACTEUR DESTINÉ À LA MISE EN OEUVRE D'UNE RÉACTION BIPHASIQUE GAZ/LIQUIDE À HAUTE PRESSION AVEC UN MILIEU MOUSSANT

(30) Priorität: 05.09.2018 EP 18192739
(43) Veröffentlichungstag der Anmeldung: 14.07.2021
(73) Patentinhaber: BASF SE, 67056 Ludwigshafen am Rhein (DE)
(72) Erfinder: BEY, Oliver, 67056 Ludwigshafen (DE); ZEHNER, Peter, 67273 Weisenheim am Berg (DE); ACKER, Michael, 67056 Ludwigshafen (DE); PACIELLO, Rocco, 67056 Ludwigshafen (DE); SCHELWIES, Mathias, 67056 Ludwigshafen (DE); HAUBNER, Martin, 67056 Ludwigshafen (DE); WEGNER, Guenter, 67056 Ludwigshafen (DE); TEBBEN, Gerd, 67056 Ludwigshafen (DE); HEYDRICH, Gunnar, 67056 Ludwigshafen (DE); SEEBER, Georg, 67056 Ludwigshafen (DE)
(74) Vertreter: BASF IP Association
(86) Internationale Anmeldenummer: PCT/EP2019/073477
(87) Internationale Veröffentlichungsnummer: WO 2020/048991

(56) Entgegenhaltungen:
- EP-A1- 1 338 333
- EP-B1- 1 231 198
- US-A- 4 000 212
- US-A1- 2018 057 437

## Beschreibung

Die Erfindung betrifft einen Reaktor zur Durchführung einer gas/flüssig-zweiphasigen Hochdruckreaktion mit einem schäumenden Medium. Die Erfindung betrifft außerdem ein Verfahren zur Durchführung einer kontinuierlichen gas/flüssig-zweiphasigen Hochdruckreaktion mit einem schäumenden Medium in dem Reaktor.

Bei zahlreichen chemischen und physikalischen Stoffumwandlungsprozessen kommt es zu einer mehr oder weniger intensiven Schaumbildung, welche die Prozessführung erschweren oder unmöglich machen. Daher sind gezielte Maßnahmen erforderlich, um den Schaum zu zerstören und/oder seine Bildung zu vermeiden oder zumindest auf ein akzeptables Maß zu mindern. Teilweise lässt sich Schaumbildung zwar durch die Strömungsführung in den Reaktoren, etwa durch Vermeidung scharfer Strömungsumlenkungen und Flüssigkeitszulauf unterhalb der Flüssigkeitsoberfläche, vermeiden oder vermindern, in vielen Fällen reichen derartige Maßnahmen aber nicht aus. Daher müssen Verfahren zur Schaumzerstörung, darunter thermische, chemische und mechanische Verfahren, ergriffen werden - eine Übersicht vermitteln Pahl et al. in Chem.-Ing.-Tech. 67 (1995), 300-312. Durch die bekannten Maßnahmen zur Schaumzerstörung wird der technische Aufwand des betreffenden Verfahrens erhöht.

Schaum kann in zwei Kategorien eingeteilt werden. Als "Blasendispersion" werden Schäume bezeichnet, bei denen der Gas Hold-Up kleiner 50% ist. Die Blasendispersion zeichnet sich dadurch aus, dass die Gasblasen etwa gleichförmig im Reaktor verteilt sind. Blasendispersionen treten auf, wenn das Medium eine hohe Viskosität aufweist und die Gasblasen nicht koaleszieren. Eine andere Form des Schaums ist der Polyederschaum, der sich in Tenside enthaltenden Flüssigkeiten bildet, wenn Gas eingebracht wird. Die tensidhaltige Flüssigkeit bildet ein dreidimensionales Netzwerk flüssiger Lamellen, welche das Gas einschließen und so Polyeder bilden. Der Polyederschaum sammelt sich an der Oberfläche des Mediums an.

Bei gas/flüssig-zweiphasigen Reaktionen ist eine intensive Durchmischung von Gasphase und Flüssigphase zur Erzielung eines hohen Umsatzes erwünscht; eine Schaumbildung ist oft nicht gänzlich zu vermeiden. Allerdings ist eine Kontamination stromabwärts gelegener Aufarbeitungsschritte oder Folgereaktionen mit Schaum oder Gasphase meist unerwünscht. Bei Hochdruckreaktionen verhindert die Schaumbildung außerdem die optimale Nutzung des Hochdruckreaktionsraums.

Die DE 10 2015 114 510 A1 beschreibt eine Vibrationsvorrichtung zur Verringerung der Schaumbildung in einem Biogasfermenter, welche im Fermenter gebildeten Schaum zersetzt.

Die DE 196 50 959 A1 beschreibt ein Verfahren zur Minderung bzw. Vermeidung einer Schaumbildung bei chemischen und physikalischen Stoffumwandlungsprozessen, bei dem in einem sich nach unten verjüngenden Reaktor mittels Gasinjektion eine Auftriebsfreistrahlumwälzung bewirkt wird.

Die EP 1 338 333 A1 beschreibt eine Reaktorkaskade aus Haupt- und Nachreaktor umfassend einen geschlossenen Hauptreaktorbehälter und einen mit dem Hauptreaktorbehälter verbundenen geschlossenen Nachreaktorbehälter. Die US 4,000,212 A beschreibt ein Verfahren zur Alkylierung eines Kohlenwasserstoffs in einem in mehrere Zonen unterteilten Reaktor. Die US 2018/057437 A1 beschreibt ein Verfahren zur Herstellung einer optisch aktiven Carbonylverbindung durch asymmetrische Hydrierung einer prochiralen α,β-ungesättigten Carbonylverbindung mit Wasserstoff.

Bei der asymmetrischen Hydrierung werden oft im Reaktionsgemisch lösliche Rhodium-Katalysatoren eingesetzt, welche in einer im Katalysezyklus durchlaufenen Form einen CO-Liganden aufweisen. Vor der asymmetrischen Hydrierung wird der Katalysator präformiert, d. h. mit einem Gasgemisch vorbehandelt, das Kohlenmonoxid und Wasserstoff enthält. Von dem so erhaltenen Katalysator wird vor dessen Einsatz in der asymmetrischen Hydrierung überschüssiges Kohlenmonoxid abgetrennt. In der industriellen Praxis wird der Katalysatorrückstand, von dem das Hydrierprodukt z. B. durch Destillation abgetrennt worden ist und der den in Schwersiedern gelösten Katalysator in seiner CO-defizitären Form enthält, der Präformierung und anschließend wieder der Hydrierreaktion zugeführt. Leider neigt der Katalysatorrückstand zum starken Schäumen, was die Präformierung und/oder anschließende Abtrennung von Kohlenmonoxid erschwert.

Der Erfindung liegt die Aufgabe zugrunde, einen Reaktor zur Durchführung einer gas/flüssig-zweiphasigen Hochdruckreaktion mit einem schäumenden Medium bereitzustellen, der so ausgelegt ist, dass der Hochdruckreaktionsraum optimal genutzt wird und eine Kontamination stromabwärts zur Hochdruckreaktion gelegener Aufarbeitungsschritte oder Folgereaktionen mit Schaum im Wesentlichen vermieden wird.

Die Aufgabe wird gelöst durch einen Reaktor zur Durchführung einer gas/flüssigzweiphasigen Hochdruckreaktion mit einem schäumenden Medium, mit
einem von einem zylindrischen, vertikal ausgerichteten länglichen Mantel, einem Boden und einer Haube gebildeten Innenraum,
wobei der Innenraum durch Einbauten in eine rückvermischte Zone und eine Zone begrenzter Rückvermischung unterteilt ist, wobei die rückvermischte Zone und die Zone begrenzter Rückvermischung vom Reaktionsgemisch nacheinander durchströmbar sind, wobei die rückvermischte Zone Mittel zum Einleiten von Gas und Flüssigkeit und einen Gasauslass aufweist sowie mindestens ein Mischorgan aufweist, welches ausgewählt ist unter einem Rührer, einer Strahldüse und Mitteln zum Einpressen des Gases, und die Zone begrenzter Rückvermischung einen Reaktionsproduktauslass aufweist,
einem ersten zylindrischen Einbauelement, welches sich im Innenraum in Längsrichtung des Reaktors erstreckt und welches die Zone begrenzter Rückvermischung von der rückvermischten Zone abgrenzt,
rückvermischungshindernden zweiten Einbauelementen in Form von Füllkörpern, strukturierten Packungen oder flüssigkeitsdurchlässigen Böden, welche in der Zone begrenzter Rückvermischung angeordnet sind, und
einem Steigrohr, dessen unteres Ende innerhalb der rückvermischten Zone angeordnet ist und dessen oberes Ende in die Zone begrenzter Rückvermischung mündet, so dass durch das Steigrohr Flüssigkeit von der rückvermischten Zone in die Zone begrenzter Rückvermischung aufsteigen kann, und
einem am unteren Ende des Steigrohrs angeordneten Einbauelement, das den Eintritt von Gas in das Steigrohr verhindert, welches Einbauelement ausgewählt ist unter einem Umlenkwehr und einem U-Rohr,
wobei die Zone begrenzter Rückvermischung von unten angeströmt wird.

Unter einer Hochdruckreaktion wird eine Reaktion verstanden, die bei einem gegenüber Umgebungsdruck erhöhtem Druck durchgeführt wird, beispielsweise bei mindestens 5 bar absolut, mindestens 20 bar absolut oder mindestens 50 bar absolut.

Der Reaktor umfasst einen von einem zylindrischen, vertikal ausgerichteten länglichen Mantel, einem Boden und einer Haube gebildeten Innenraum. Das Verhältnis von Länge zu Durchmesser des Mantels beträgt üblicherweise 2:1 bis 100:1, bevorzugt 5:1 bis 100:1, besonders bevorzugt 5:1 bis 50:1, ganz besonders bevorzugt 5:1 bis 30:1.

Der Innenraum des Reaktors ist durch Einbauten in eine rückvermischte Zone und eine Zone begrenzter Rückvermischung unterteilt. Die rückvermischte Zone und die Zone begrenzter Rückvermischung sind vom Reaktionsgemisch nacheinander durchströmbar. Die Zone begrenzter Rückvermischung wird von unten angeströmt, vorzugsweise durch einen Durchlass wie einer Strahldüse am Boden der Zone begrenzter Rückvermischung. Ein erstes zylindrisches Einbauelement erstreckt sich im Innenraum in Längsrichtung des Reaktors und grenzt die Zone begrenzter Rückvermischung von der rückvermischten Zone ab.

Vorzugsweise liegt das Volumenverhältnis von rückvermischter Zone zur Zone begrenzter Rückvermischung im Bereich von 0,25:1 bis 4:1, besonders bevorzugt im Bereich von 0,3:1 bis 3:1. Ein Reaktor mit einem Volumenverhältnis in diesem Bereich erlaubt eine optimale Ausnutzung des Reaktorraums.

Wird im erfindungsgemäßen Reaktor eine gas/flüssig-zweiphasige Hochdruckreaktion durchgeführt, sammelt sich Gas im oberen Teil der rückvermischten Zone und bildet eine Gasphase, während im unteren Teil der rückvermischten Zone eine Flüssigphase vorliegt. In der rückvermischten Zone gebildeter Schaum schwimmt üblicherweise auf der Flüssigphase auf.

Die Einspeisung von Gas und Flüssigkeit erfolgt an einer beliebigen Stelle der rückvermischten Zone.

Die rückvermischte Zone weist wenigstens ein Mischorgan auf, welches ausgewählt ist unter einem Rührer, einer Strahldüse und Mitteln zum Einpressen des Gases. Hierdurch werden das Gas und die Flüssigkeit miteinander in intensiven Kontakt gebracht.

In einer Ausführungsform ist das Mischorgan ein Rührer, z. B. ein Propeller-Rührer.

In einer bevorzugten Ausführungsform erfolgt die Einspeisung von Gas und Flüssigkeit so, dass gleichzeitig eine Durchmischung der rückvermischten Zone bewirkt wird. In diesem Fall sind die Mittel zum Einleiten von Flüssigkeit als Mischorgan, nämlich als Strahldüse, und/oder die Mittel zum Einleiten von Gas als Mischorgan, nämlich als Mittel zum Einpressen des Gases ausgebildet.

Vorzugsweise erfolgt das Einleiten der Flüssigkeit durch eine Strahldüse. Die Einleitung durch eine Strahldüse kann oberhalb oder unterhalb des Trennspiegels von Gas und Flüssigkeit erfolgen, insbesondere durch eine am Boden der rückvermischten Zone angeordnete, nach oben gerichtete Strahldüse.

Die Strahldüse kann als Einstoff- oder Zweistoffdüse ausgelegt werden. Bei der Einstoffdüse wird nur eine Flüssigkeit eingedüst. Vorteilhaft bei dieser Ausgestaltung ist der einfache Aufbau einer solchen Einstoffdüse. Bei der Zweistoffdüse werden das Gas und die Flüssigkeit zusammen zugeführt und dispergiert.

Um das Gas zusammen mit der Flüssigkeit gemeinsam einzuleiten, kann die Strahldüse als Mischdüse ausgebildet sein, z. B. eine Strahlsauger-Mischdüse (Flüssigkeitsstrahl-Ejektor). Der Begriff Mischdüse bezeichnet in üblicher Weise ein sich in Strömungsrichtung verjüngendes Rohr. Der austretende schnelle Strahl erzeugt in einem die Düse umgebenden Ansaugraum einen Unterdruck. Dadurch kann Gas angesaugt und infolge des Impulsaustausches in der Flüssigkeit dispergiert und gemeinsam mit dieser in die rückvermischte Zone entlassen werden.

In einer weiteren Ausführungsform umfasst das Mischorgan Mittel zum Einpressen des Gases in die Flüssigkeit. Geeignete Mittel zum Einpressen des Gases sind z. B. ein Kompressor zum Ansaugen und Komprimieren des Gases oberhalb des Trennspiegels bzw. von Frischgas und Düsen zum Einpressen des komprimierten Gases unterhalb des Trennspiegels.

Die rückvermischte Zone ist geeigneter Weise als Auftriebsfreistrahlreaktor ausgebildet, der durch eine punkt- oder linienförmige Fluidinjektion eine großräumige Umwälzung des Inhalts der rückvermischten Zone erlaubt. In einer Ausführungsform ist die rückvermischte Zone im Wesentlichen frei von Einbauten wie Leitblechen, Rührer und dergleichen. Dies erlaubt eine größere Flexibilität beim Einstellen des Flüssigkeitsstandes.

Vorzugsweise ist die rückvermischte Zone als Schlaufenreaktor ausgebildet. Beispiele für Schlaufenreaktoren sind Rohrreaktoren mit internen und externen Schlaufen. Solche Reaktoren sind beispielsweise in Ullmanns Enzyklopädie näher beschrieben (Ullmann's Encyclopedia of Industrial Chemistry, Verlag Chemie, Electronic Release 2008, 7th Edition, Kapitel "Stirred Tank and Loop Reactors" und Kapitel "Bubble Columns").

Der Schlaufenreaktor weist im Allgemeinen einen externen Kreislauf (externe Schlaufen) auf. Bei einem Schlaufenreaktor mit externem Kreislauf befindet sich in der Regel ein Abzug an beliebiger Stelle der rückvermischten Zone, vorzugsweise im unteren Bereich der rückvermischten Zone, über den das Reaktionsgemisch in einem äußeren Kreislauf mittels eines Förderorgans der Injektionsdüse wieder zugeführt wird. Das Förderorgan ist vorzugsweise eine Pumpe, weshalb der externe Kreislauf üblicherweise als Umpumpkreislauf bezeichnet wird.

Beispiele für Pumpen sind Kreiselpumpen oder Rotationskolbenpumpen, wie Drehkolbenpumpen, Drehschieberpumpen, Kreiskolbenpumpen oder Zahnradpumpen. Besonders bevorzugt werden Kreiselpumpen als Förderorgan verwendet.

Vorzugsweise befindet sich im externen Kreislauf des Schlaufenreaktors ein Wärmetauscher. Ein so ausgestalteter Schlaufenreaktor wird im Rahmen dieser Erfindung als Schlaufenreaktor mit externem Wärmetauscher bezeichnet.

Der Wärmetauscher ist beispielsweise ein Rohrbündelwärmetauscher, Doppelrohrwärmetauscher, Plattenwärmetauscher oder Spiralwärmetauscher. Bei Auslegungsdrücken für den Reaktor unter 100 bar wird bevorzugt ein Rohrbündelwärmetauscher verwendet, bei höheren Drücken wird bevorzugt ein oder mehrere hintereinander geschaltete Doppelrohrwärmetauscher verwendet.

Der Schlaufenreaktor mit externem Wärmetauscher wird dabei üblicherweise so betrieben, dass ein Teil des Reaktionsgemisches aus der rückvermischten Zone durch den externen Umpumpkreislauf, in dem sich der externe Wärmetauscher befindet, gefördert wird, wobei das durch den Wärmetauscher geförderte Reaktionsgemisch gekühlt wird. Durch das externe Umpumpen wird das Reaktionsgemisch in der ersten Reaktionsstufe in der Regel stark durchmischt und umgewälzt, so dass die Verweilzeit in der ersten Stufe üblicherweise der eines vollständig rückvermischten Rührkessels (CSTR) entspricht. Das Reaktionsgemisch wird schließlich mittels der Injektionsdüse wieder der rückvermischten Zone zugeführt. Üblicherweise werden frisches Gas und frische Flüssigkeit in den Umpumpkreislauf dosiert und zusammen mit dem bereits im Umpumpkreislauf befindlichen Strom als Reaktionsgemisch der rückvermischten Zone zugeführt.

Die rückvermischte Zone weist einen Gasauslass auf. Über diesen kann nicht umgesetztes Gas abgezogen werden. Der Gasauslass befindet sich vorzugsweise am oberen Ende des zylindrischen Mantels. Vorteilhafterweise ist der Reaktor so ausgestaltet, dass das abgezogene, nicht umgesetzte Gas zumindest teilweise über die Injektionsdüse wieder in das Reaktionsgemisch in der rückvermischten Zone eingespeist werden kann. Hierfür kann das nicht umgesetzte Gas aus dem Gasauslass über eine externe Gasleitung zur Injektionsdüse geführt werden.

In der rückvermischten Zone kann bei starker Schaumbildung und/oder hohem Flüssigkeitsstand Schaum von der Flüssigphase bis zum Gasauslass aufsteigen. Eine Kontamination des Gasauslasses und daran angeschlossener Leitungen mit Schaum ist üblicherweise unerwünscht. Das Ausmaß der Schaumbildung ist in der Regel variabel und schwer vorhersagbar. Es ist in der Regel erforderlich, die Anwesenheit von Schaum im Bereich des Gasauslasses zu verhindern.

Vorzugsweise umfasst der Reaktor in einer Ausführungsform mindestens ein drittes Einbauelement, welches in der oberen Hälfte der rückvermischten Zone angeordnet ist und eine Oberfläche aufweist, welche die Koaleszenzneigung schäumender Medien unterstützt. Geeignete Einbauelemente, welche die Koaleszenzneigung schäumender Medien unterstützen, umfassen Elemente zur chemischen, thermischen oder mechanischen Schaumzerstörung. Eine Übersicht vermitteln Pahl et al. in Chem.-Ing.-Tech. 67 (1995), 300-312. In einer Ausführungsform umfasst der Reaktor mechanische Schaumzerstörer, wie beispielsweise rotierende Elemente, oder Einbauten zur Beregnung mit arteigener Flüssigkeit.

Der Reaktor umfasst ein Steigrohr. Das Steigrohr ist so angeordnet, dass sein unteres Ende während der gas/flüssig-zweiphasigen Hochdruckreaktion in die Flüssigphase eintaucht. Durch den bei der Reaktion anfallenden Schaum ist die Grenze zwischen Flüssig- und Gasphase in der rückvermischten Zone üblicherweise nicht scharf definierbar. Das Steigrohr taucht geeigneter Weise so tief in die Flüssigkeit ein, dass während der Reaktion im Wesentlichen kein Schaum ins Steigrohr eindringen kann. So steigt Flüssigkeit von der rückvermischten Zone in die Zone begrenzter Rückvermischung auf, ohne dass signifikante Mengen an Schaum mitgeführt werden. Dadurch wird eine Kontamination stromabwärts gelegener Aufarbeitungsschritte oder Folgereaktionen mit Schaum im Wesentlichen vermieden. Dieser Effekt ist bei auf der Flüssigphase aufschwimmenden Schäumen, wie Polyederschäumen, besonders ausgeprägt.

Das untere Ende des Steigrohrs ist beabstandet zum Boden angeordnet. Im Betrieb weist das untere Ende des Steigrohrs einen Abstand zum Boden auf, der im Bereich von 10 bis 95%, besonders bevorzugt 30 bis 90% und ganz besonders bevorzugt 70 bis 80% der Höhe des Flüssigkeitsstandes liegt.

Das Steigrohr weist üblicherweise einen Durchmesser auf, der im Bereich von 1 bis 90%, vorzugsweise 2 bis 50%, ganz besonders bevorzugt 5 bis 20% des Durchmessers der Zone begrenzter Rückvermischung liegt.

Erfindungsgemäß umfasst der Reaktor ein am unteren Ende des Steigrohrs angeordnetes viertes Einbauelement, das den Eintritt von Gasblasen in das Steigrohr im Wesentlichen verhindert. Insbesondere verhindern die Form und Anordnung des vierten Einbauelements im Wesentlichen, dass im Reaktionsgemisch aufsteigende Gasblasen in das Steigrohr eintreten. Das vierte Einbauelement ist ausgewählt unter einem Umlenkwehr und einem U-Rohr, besonders bevorzugt ist ein Umlenkwehr.

Die Rückvermischung in der Zone begrenzter Rückvermischung wird durch rückvermischungshindernde zweite Einbauelemente begrenzt. Durch den Einbau solcher Vorrichtungen werden in der Regel die Zirkulation und damit die Rückvermischung von Gas- und Flüssigkeit eingeschränkt. Die Verweilzeitverteilung in der Zone begrenzter Rückvermischung ist der eines Rohrreaktors angenähert. Durch diese definierte Flüssigkeitsverweilzeit wird ein hoher Umsatz bei der Hochdruckreaktion erreicht.

Vorzugsweise ist der Anteil der Gasphase am Reaktionsgemisch in der Zone begrenzter Rückvermischung im Vergleich zur rückvermischten Zone vermindert. Dieser Effekt kann beispielsweise durch die Verwendung eines Steigrohrs und ggf. eines vierten Einbauelements, das den Eintritt von Gas in das Steigrohr im Wesentlichen verhindert, erzielt werden. Durch die Verringerung einer diskreten Gasphase in der Zone begrenzter Rückvermischung kann der Flüssigkeits-Holdup der Zone begrenzter Rückvermischung vergrößert und die Verweilzeit der Flüssigphase in der Zone begrenzter Rückvermischung erhöht werden. Da Hochdruckreaktionen im Wesentlichen in der Flüssigphase erfolgen, wird der Reaktionsraum auf diese Weise optimal genutzt.

Die Begrenzung der Rückvermischung in der Zone begrenzter Rückvermischung wird durch den Einbau von Füllkörpern, strukturierten Packungen oder flüssigkeitsdurchlässigen Böden realisiert. In einer bevorzugten Ausführungsform erfolgt die Begrenzung der Rückvermischung durch den Einbau von mehreren, fest angeordneten, flüssigkeitsdurchlässigen Böden in der Zone begrenzter Rückvermischung. Es entstehen zwischen den einzelnen Böden somit Einzelsegmente ("Kompartimente") mit definierten Reaktionsvolumina. Jedes der Einzelsegmente wirkt in der Regel dabei wie ein einzelner, rückvermischter Rührkesselreaktor. Mit zunehmender Anzahl von Einzelsegmenten hintereinander nähert sich die Verweilzeitverteilung einer derartigen Kaskade in der Regel der Verweilzeit eines Rohrreaktors an.

Die Anzahl der so gebildeten Einzelsegmente beträgt vorzugsweise 2 bis 20, besonders bevorzugt 2 bis 10, insbesondere bevorzugt 3 bis 6. Das Volumen der gebildeten Einzelsegmente ist üblicherweise im Wesentlichen gleich. Besonders bevorzugt sind die Böden als Lochbleche gestaltet.

In einer weiteren Ausführungsform erfolgt die Begrenzung der Rückvermischung durch den Einbau von Füllkörpern. Die Füllkörper können unterschiedliche Formen aufweisen und sind meist etwa 2 bis 15 mm groß. Bekannte Beispiele sind kugelförmige und zylinderförmige Vollkörper, Raschig-Ringe (Hohlzylinder), Pall-Ringe, Hiflow-Ringe, Intalox-Sattel und dergleichen. Vorzugsweise sind die Füllkörper Vollkörper. Die Füllkörper können geordnet, aber auch regellos (als Schüttung) in die Zone begrenzter Rückvermischung eingebracht werden. Als Material können Glas, Keramik, Metall und Kunststoffe verwendet werden.

In einer weiteren Ausführungsform erfolgt die Begrenzung der Rückvermischung durch den Einbau von strukturierten Packungen. Strukturierte Packungen sind eine Weiterentwicklung der geordneten Füllkörper. Sie weisen eine regelmäßig geformte Struktur auf. Es gibt verschiedene Ausführungen von Packungen, wie Gewebe- oder Blechpackungen. Als Material können Metall, Kunststoff, Glas und Keramik verwendet werden.

Vorzugsweise ist das erste Einbauelement konzentrisch zum Mantel angeordnet, so dass die Zone begrenzter Rückvermischung einen kreisförmigen horizontalen Querschnitt aufweist. Das Verhältnis von Länge zu Durchmesser der Zone begrenzter Rückvermischung beträgt üblicherweise 2:1 bis 100:1, bevorzugt 5:1 bis 50:1, besonders bevorzugt 7:1 bis 25:1.

Es wird ferner ein Verfahren zur Durchführung einer kontinuierlichen gas/flüssigzweiphasigen Hochdruckreaktion, bei dem man in einen Reaktor nach einem der vorhergehenden Ansprüche ein Gas und eine Flüssigkeit in die rückvermischte Zone einführt, Flüssigkeit durch das Steigrohr von der rückvermischten Zone in die Zone begrenzter Rückvermischung aufsteigen lässt, nicht umgesetztes Gas über den Gasauslass zumindest teilweise ausführt, und ein Reaktionsprodukt am Reaktionsproduktauslass abzieht.

Vorteilhafterweise wird das nicht umgesetzte Gas zumindest teilweise über die Injektionsdüse wieder in das Reaktionsgemisch in der rückvermischten Zone eingespeist, beispielsweise über eine externe Gasleitung.

Geeigneter Weise herrscht in der rückvermischten Zone ein höherer Druck als am Reaktionsproduktauslass, um den in der Zone begrenzter Rückvermischung herrschenden hydrostatischen Druck und den beim Durchströmen der rückvermischungshindernden zweiten Einbauelemente anfallenden Druckverlust zu überwinden. Durch den höheren Druck in der rückvermischten Zone steigt Flüssigkeit von der rückvermischten Zone in die Zone begrenzter Rückvermischung auf. Das erste zylindrische Einbauelement und die rückvermischungshindernden zweiten Einbauelemente müssen entsprechend dieses Differenzdruckes ausgelegt sein.

In einer Ausführungsform handelt es sich bei dem Verfahren um ein Verfahren zur Präformierung eines homogenen Rhodium-Hydrierkatalysators, der mindestens einen CO-Liganden enthält. Es handelt sich also um ein Verfahren zur Behandlung eines CO-defizienten Rhodium-Hydrierkatalysators mit einem Gasgemisch, das Kohlenmonoxid und Wasserstoff enthält. Dabei umfasst die Flüssigkeit einen gelösten CO-defizienten Rhodium-Hydrierkatalysator und das Gas enthält Wasserstoff und Kohlenmonoxid, wobei aus der Reaktion des CO-defizienten Rhodium-Hydrierkatalysators mit dem Gas ein hydrieraktiver Rhodium-Hydrierkatalysator erhalten wird.

Das Reaktionsprodukt der Präformierung, das den hydrieraktiven Rhodium-Hydrierkatalysator, kann dann zusammen mit einem zu hydrierenden Substrat einer asymmetrischen Hydrierungsreaktion zugeführt werden, wobei ein Hydrierreaktionsgemisch erhalten wird. Nach Abtrennen des Hydrierprodukts führt man den Rückstand mit CO-defizientem Rhodium-Hydrierkatalysator wieder der Präformierung zu. Das Hydrierprodukt lässt sich durch dem Fachmann an sich bekannte Verfahren, wie z. B. durch Destillation und/oder Entspannungsverdampfung, aus dem Hydriergemisch abtrennen, wobei der hydrieraktive Rhodium-Hydrierkatalysator CO verliert und ein CO-defizienter Rhodium-Hydrierkatalysator zurück bleibt.

In einer Ausführungsform ist das zu hydrierende Substrat cis-Citral. Das Hydrierprodukt des cis-Citral ist R-Citronellal.

Die zur Hydrierung eingesetzten Rhodium-Katalysatoren weisen zumindest in einer im Katalysezyklus durchlaufenen Form oder in einer dem eigentlichen Katalysezyklus vorgeschalteten Vorform mindestens einen CO-Liganden auf, wobei es unerheblich ist, ob diese mindestens einen CO-Liganden aufweisende Katalysatorform die tatsächliche katalytisch aktive Katalysatorform darstellt. Um die CO-Liganden aufweisende Katalysatorformen zu stabilisieren, kann es vorteilhaft sein, dem Reaktionsgemisch während der Hydrierung zusätzlich Kohlenmonoxid zuzuführen.

Der Rhodium-Katalysator weist üblicherweise mindestens einen optisch aktiven Liganden auf. Derartige Katalysatoren sind beispielsweise erhältlich durch Reaktion einer geeigneten, im Hydrierungsgemisch löslichen Rhodium-Verbindung mit einem optisch aktiven Liganden, der mindestens ein Phosphor- und/oder Arsenatom aufweist. Beispiele für einsetzbare Rhodium-Verbindungen sind: RhCl₃, Rh(OAc)₃, [Rh(cod)Cl]₂, Rh(CO)₂acac, [Rh(cod)OH]₂, [Rh(cod)OMe]₂, Rh₄(CO)₁₂, Rh₆(CO)₁₆, wobei "acac" für einen Acetylacetonat- und "cod" für einen Cyclooctadien-Liganden steht.

Die genannten Rhodiumverbindungen werden mit einer weiteren Verbindung in Kontakt gebracht, die optisch aktiv, bevorzugt im wesentlichen enantiomerenrein ist (d. h. einen Enantiomerenüberschuss von mindestens etwa 99% aufweist) und mindestens ein Phosphor und/oder Arsenatom, bevorzugt mindestens ein Phosphoratom aufweist. Diese als chiraler Ligand zu bezeichnende Verbindung bildet mit der eingesetzten Rhodium-Verbindung den Rhodium-Katalysator.

Insbesondere bevorzugt sind solche chiralen Liganden die zwei Phosphoratome aufweisen und mit Rhodium Chelatkomplexe bilden.

Als chirale Liganden eignen sich im Rahmen der vorliegenden Erfindung solche Verbindungen, wie sie beispielsweise in: I. Ojima (Hrsg.), Catalytic Asymmetric Synthesis, Wiley-VCh, 2. Auflage, 2000 oder in E. N. Jacobsen, A. Pfaltz, H. Yamamoto (Hrsg.), Comprehensive Asymmetric Catalysis, 2000, Springer oder in W. Tang, X. Zhang, Chem. Rev. 2003, 103, 3029-3069 beschrieben sind.

Bevorzugte Liganden sind chirale zweizähnige Bisphosphin-Liganden, insbesondere solche der allgemeinen Formeln (I) bis (III) 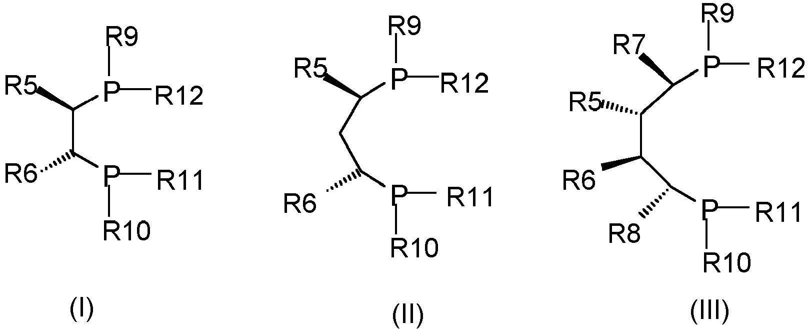 worin
R⁵, R⁶ jeweils unabhängig voneinander für einen unverzweigten, verzweigten oder cyclischen Kohlenwasserstoffrest mit 1 bis 20 Kohlenstoffatomen stehen, der gesättigt ist oder eine oder mehrere, in der Regel 1 bis etwa 4, nicht konjugierte, ethylenische Doppelbindungen aufweisen kann und der unsubstituiert ist oder einen oder mehrere, in der Regel 1 bis 4, gleiche oder verschiedene Substituenten trägt, die ausgewählt sind unter OR¹³, NR¹⁴R¹⁵, Halogen, C₆-C₁₀-Aryl und C₃-C₉-Hetaryl, oder
R⁵ und R⁶ gemeinsam eine 2 bis 10-gliedrige Alkylengruppe oder eine 3 bis 10-gliedrige Cycloalkylengruppe bedeuten kann, worin 1 , 2, 3 oder 4 nicht benachbarte CH-Gruppen durch O oder N-R¹³ ersetzt sein können, wobei die Alkylengruppe und die Cycloalkylengruppe gesättigt sind oder eine oder zwei, nicht konjugierte ethylenische Doppelbindungen aufweisen, und wobei die Alkylengruppe und die Cycloalkylengruppe unsubstituiert sind oder einen oder mehrere gleiche oder verschiedene Substituenten tragen, die ausgewählt sind unter C₁-C₄-Alkyl;
R⁷, R⁸ jeweils unabhängig voneinander Wasserstoff oder geradkettiges oder verzweigtes C₁-C₄-Alkyl bedeuten und
R⁹, R¹⁰, R¹¹, R¹² gleich oder verschieden sind und für C₆-C₁₀-Aryl stehen, das unsubstituiert ist oder einen oder mehrere Substituenten trägt, die ausgewählt sind unter C₁-C₆-Alkyl, C₃-C₆-Cycloalkyl, C₆-C₁₀-Aryl, C₁-C₆-Alkoxy und Amino;
R¹³, R¹⁴, R¹⁵ jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei R¹⁴ und R¹⁵ auch gemeinsam eine Alkylenkette mit 2 bis 5 Kohlenstoffatomen, die durch N oder O unterbrochen sein kann, bedeuten können.

Im Hinblick auf die Formeln (I), (II) und (III) haben die Variablen insbesondere die folgende Bedeutung:
R⁵, R⁶ stehen jeweils unabhängig voneinander für C₁-C₄-Alkyl oder
R⁵ und R⁶ stehen gemeinsam für einen C₃-C₅-Alkandiyl-Rest, C₃-C₇-Alkendiyl-Rest, C₅-C₇-Cycloalkandiyl-Rest oder einen C₅-C₇-Cycloalkendiyl-Rest, wobei die vier vorgenannten Reste unsubstituiert sind oder einen oder mehrere gleiche oder verschiedene Substituenten tragen, die ausgewählt sind unter C₁-C₄-Alkyl;
R⁷, R⁸ stehen jeweils unabhängig voneinander Wasserstoff oder C₁-C₄-Alkyl;
R⁹, R¹⁰, R¹¹, R¹² stehen jeweils für Phenyl.

Aufgrund ihrer leichten Verfügbarkeit besonders bevorzugte chirale, zweizähnige Bisphosphin-Liganden sind unter der Bezeichnung "Chiraphos" erhältliche Verbindungen der Formel:

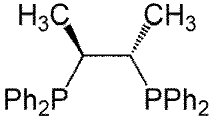

Die chiralen Liganden werden vorteilhaft in einer Menge von etwa 0,9 bis etwa 10 mol, bevorzugt etwa 1 bis etwa 4 mol pro mol eingesetzter Rhodium-Verbindung eingesetzt. Geeigneter Weise wird der optisch aktive Rhodium-Katalysator durch Umsetzung einer achiralen Rhodiumverbindung und mit einem chiralen, zweizähnigen Bisphosphin-Liganden in-situ generiert. In diesem Zusammenhang bedeutet der Ausdruck "in-situ", dass der Katalysator direkt vor der Hydrierung generiert wird.

Es wurde gefunden, dass die Gegenwart einzähniger Liganden die Aktivität des Katalysators erhöhen kann. In einer bevorzugten Ausführungsform des erfindungsgemäßen Verfahrens werden Verbindungen der Formel (IV) 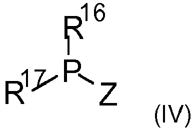 eingesetzt, beispielsweise in der in den Reaktor eingeführten Flüssigkeit, worin Z in Formel (IV) für eine Gruppe CHR¹⁸R¹⁹ steht und worin die Variablen R¹⁶, R¹⁷, R¹⁸, R¹⁹ unabhängig voneinander und insbesondere gemeinsam die folgenden Bedeutungen aufweisen:
- R¹⁶, R¹⁷:: sind gleich oder verschieden und stehen für Phenyl, das unsubstituiert ist oder 1, 2 oder 3 Substituenten trägt, die ausgewählt sind unter Methyl und Methoxy, wobei R¹⁶ und R¹⁷ jeweils insbesondere für unsubstituiertes Phenyl stehen;
- R¹⁸: steht für C₁- bis C₄-Alkyl, insbesondere für Methyl;
- R¹⁹: steht für C₁- bis C₄-Alkyl, das eine Gruppe P(=O)R^{19a}R^{19b} trägt, wobei und insbesondere eine Gruppe CH₂-P(=O)R^{19a}R^{19b} oder CH(CH₃)-P(=O)R^{19a}R^{19b} bedeutet;
wobei
- R^{19a}, R^{19b}:: gleich oder verschieden sind und für Phenyl stehen, das unsubstituiert ist oder 1, 2 oder 3 Substituenten trägt, die ausgewählt sind unter Methyl und Methoxy, wobei besonders bevorzugt R^{19a} und R^{19b} jeweils für unsubstituiertes Phenyl stehen.

In dieser bevorzugten Ausführungsform des erfindungsgemäßen Verfahrens wird besonders bevorzugt eine Verbindung der Formel (IV) eingesetzt, worin
- R16, R¹⁷:: für unsubstituiertes Phenyl stehen;
- R¹⁸: für Methyl steht;
- R¹⁹: eine Gruppe CH(CH₃)-P(=O)R^{19a}R^{19b} bedeutet, worin R^{19a} und R^{19b} jeweils für unsubstituiertes Phenyl stehen.

Hierbei handelt es sich um die Verbindung (2-(Diphenylphosphoryl)-1-methylpropyl)-diphenylphosphan (Chiraphosmonooxid), einschließlich des (R,R)-Enantiomers (= (R,R)-Chiraphosmonooxid) und des (S,S)-Enantiomers (= (S,S)-Chiraphosmonooxid) sowie Gemischen aus (R,R)-Chiraphosmonooxid und (S,S)-Chiraphosmonooxid.

Falls die Reste R¹⁸ und R¹⁹ in der allgemeinen Formel (IV) unterschiedliche Bedeutung haben, kann das Kohlenstoffatom, welches die Reste R¹⁸ und R¹⁹ trägt, eine (R)- oder (S)-Konfiguration aufweisen. Diese Verbindungen der allgemeinen Formel (IV) können als reine (R)- oder reine (S)-Stereoisomere oder als Mischungen davon vorliegen. In der Regel wird man in diesen Fällen die reinen (R)- und (S)-Stereoisomere einsetzen, wobei auch etwaige Stereoisomerengemische für den Einsatz in dem vorliegenden Verfahren geeignet sind.

Unter einem reinen Stereoisomeren versteht man hier und im Folgenden chirale Substanzen, die bezüglich des gewünschten Stereoisomeren einen Enantiomerenüberschuss (ee) von wenigstens 80%ee, insbesondere wenigstens 90%ee und speziell wenigstens 95%ee aufweisen.

Insbesondere wird als chiraler Ligand Chiraphos verwendet und als einzähnig bindende Verbindung (2-(Diphenylphosphoryl)-1-methylpropyl)-diphenylphosphan (Chiraphosmonooxid). Beispielsweise wird als chiraler Ligand R-Chiraphos verwendet und als einzähnig bindende Verbindung (*R,R*)-Chiraphosmonooxid und/oder (*S,S*)-Chiraphosmonooxid. Alternativ wird als chiraler Ligand S-Chiraphos verwendet und als einzähnig bindende Verbindung (*R,R*)-Chiraphosmonooxid und/oder (*S,S*)-Chiraphosmonooxid.

Die Verbindung der Formel (IV) wird erfindungsgemäß in der Regel in einer Menge von 0,01 bis 1 Mol, bevorzugt 0,02 bis 0,8 Mol, besonders bevorzugt 0,03 bis 0,7 Mol und insbesondere in einer Menge von 0,04 bis 0,6 Mol pro Mol Rhodium einsetzt.

Weitere Ausführungsformen des Hydrierkatalysators und des einzähnigen Liganden sind in US 2018/0057437 A1, WO 2006/040096 A1 und WO 2008/132057 A1 beschrieben.

Bei der Präformierung des Rhodium-Katalysators löst man üblicherweise die gewählte Rhodium-Verbindung und den gewählten chiralen Liganden in einem geeigneten, unter den Reaktionsbedingungen inerten Lösungsmittel bzw. Lösungsmedium wie beispielsweise Ether, Tetrahydrofuran, Methyltetrahydrofuran, Toluol, Xylole, Chlorbenzol, Octadecanol, Biphenylether, Texanol, Marlotherm, Oxoöl 9N (Hydroformylierungsprodukte aus isomeren Octenen, BASF Aktiengesellschaft), Citronellal und dergleichen. Als Lösungsmedium können auch das Hydrierprodukt oder bei der Umsetzung eventuell anfallende hochsiedende Nebenprodukte dienen. Der resultierenden Lösung wird im erfindungsgemäßen Reaktor bei einem Druck von üblicherweise etwa 5 bis etwa 350 bar, bevorzugt von etwa 20 bis etwa 200 bar und besonders bevorzugt von etwa 50 bis etwa 100 bar ein Gasgemisch aufgepresst, das Wasserstoff und Kohlenmonoxid enthält. Bevorzugt setzt man zur Präformierung ein Gasgemisch ein, das etwa
30 bis 99 Vol.-% Wasserstoff,
1 bis 70 Vol.-% Kohlenmonoxid und
0 bis 5 Vol.-% weiterer Gase enthält, wobei sich die Angaben in Vol.-% zu 100 Vol.-% addieren.

Ein zur Präformierung insbesondere bevorzugtes Gasgemisch ist sogenanntes Synthesegas, das üblicherweise zu etwa 35 bis 55 Vol-% aus Kohlenmonoxid neben Wasserstoff und Spuren weiterer Gase besteht.

Die Präformierung des Katalysators wird üblicherweise bei Temperaturen von etwa 25 °C bis etwa 100 °C, bevorzugt bei etwa 40 °C bis etwa 80 °C durchgeführt. Die Präformierung ist üblicherweise nach etwa 1 h bis etwa 24 h, oft nach etwa 1 bis etwa 12 h abgeschlossen. Im Anschluss an die Präformierung führt man vorzugsweise die asymmetrische Hydrierung eines gewählten Substrats durch. Nach vorangegangener Präformierung lässt sich das gewählte Substrat in der Regel mit gutem Erfolg mit oder ohne Zuführung von zusätzlichem Kohlenmonoxid durchführen. Vorteilhafterweise führt man eine Präformierung wie beschrieben durch und setzt dem Reaktionsgemisch während der asymmetrischen Hydrierung zusätzliches Kohlenmonoxid zu.

In dieser Ausführungsform wird die genannte Präformierung der Katalysator-Vorstufe mit einem Gasgemisch umfassend 20 bis 90 Vol.-% Kohlenmonoxid, 10 bis 80 Vol-% Wasserstoff und 0 bis 5 Vol.-% weiterer Gase, wobei sich die genannten Volumenanteile zu 100 Vol.-% ergänzen, bei einem Druck von 5 bis 100 bar durchführt. Zusätzlich wird von dem so erhaltenen Katalysator vor Einsatz in der asymmetrischen Hydrierung überschüssiges Kohlenmonoxid abgetrennt. Unter dem Begriff überschüssiges Kohlenmonoxid ist dabei solches Kohlenmonoxid zu verstehen, das in dem erhaltenen Reaktionsgemisch in gasförmiger oder gelöster Form enthalten ist und nicht an den Rhodium-Katalysator bzw. dessen Vorstufe gebunden ist. Demgemäß entfernt man das überschüssige, nicht an den Katalysator gebundene Kohlenmonoxid zumindest weitgehend, d. h. in einem Ausmaß, dass sich eventuelle Restmengen gelösten Kohlenmonoxids in der folgenden Hydrierung nicht störend bemerkbar machen. Dies ist üblicherweise gewährleistet, wenn etwa 90%, bevorzugt etwa 95% oder mehr des zur Präformierung eingesetzten Kohlenmonoxids abgetrennt werden. Bevorzugt entfernt man das überschüssige Kohlenmonoxid vollständig von dem durch Präformierung erhaltenen Katalysator.

Die Abtrennung des überschüssigen Kohlenmonoxids vom erhaltenen Katalysator bzw. vom den Katalysator enthaltenden Reaktionsgemisch kann auf verschiedenen Wegen erfolgen. Bevorzugt entspannt man den Katalysator bzw. das durch Präformierung erhaltene, den Katalysator enthaltende Gemisch auf einen Druck von bis zu etwa 5 bar (absolut), vorzugsweise, speziell, bei Durchführung der Präformierung im Druckbereich von 5 bis 10 bar, auf einen Druck von weniger als 5 bar (absolut), bevorzugt auf einen Druck im Bereich von etwa 1 bar bis etwa 5 bar, vorzugsweise 1 bis weniger als 5 bar, besonders bevorzugt auf einen Druck im Bereich von 1 bis 3 bar, ganz besonders bevorzugt auf einen Druck im Bereich von etwa 1 bis etwa 2 bar, insbesondere bevorzugt auf Normaldruck, so dass gasförmiges, nicht gebundenes Kohlenmonoxid aus dem Produkt der Präformierung entweicht. Die vorstehend genannte Entspannung des präformierten Katalysators kann beispielsweise unter Einsatz eines Hochdruckabscheiders, wie er dem Fachmann an sich bekannt ist, erfolgen. Derartige Abscheider, bei denen die Flüssigkeit in der kontinuierlichen Phase ist, sind beispielsweise beschrieben in: Perry's Chemical Engineers' Handbook, 1997, 7. Aufl., McGraw-Hill, S. 14.95 und 14.96; die Verhinderung eines möglichen Tropfenmitrisses ist auf den Seiten 14.87 bis 14.90 beschrieben. Die Entspannung des präformierten Katalysators kann einstufig oder zweistufig bis zum Erreichen des gewünschten Druckes im Bereich von 1 bar bis etwa 5 bar erfolgen, wobei die Temperatur üblicherweise auf 10 bis 40°C sinkt. Alternativ kann die Abtrennung überschüssigen Kohlenmonoxids durch sogenanntes Strippen des Katalysators bzw. des den Katalysator enthaltenden Gemischs mit einem Gas, vorteilhaft mit einem unter den Reaktionsbedingungen inerten Gas, erreicht werden. Unter dem Begriff Strippen versteht der Fachmann dabei das Einleiten eines Gases in den Katalysator bzw. das den Katalysator enthaltende Reaktionsgemisch wie beispielsweise in W. R. A. Vauck, H. A. Müller, Grundoperationen chemischer Verfahrenstechnik, Deutscher Verlag für Grundstoffchemie Leipzig, Stuttgart, 10 Auflage, 1984, Seite 800, beschrieben. Als geeignete inerte Gase seien hierfür beispielhaft genannt: Wasserstoff, Helium, Neon, Argon, Xenon, Stickstoff und/oder CO2, bevorzugt Wasserstoff, Stickstoff, Argon.

Falls dem Reaktionssystem Kohlenmonoxid zugeführt wird, kann die Zuführung auf verschiedene Weise vorgenommen werden: So kann das Kohlenmonoxid beispielsweise dem zur asymmetrischen Hydrierung eingesetzten Wasserstoff beigemischt werden oder auch direkt gasförmig in die Reaktionslösung eindosiert werden. Bevorzugt wird das Kohlenmonoxid dem zur asymmetrischen Hydrierung eingesetzten Wasserstoff beigemischt.

Bevorzugt wird die asymmetrische Hydrierung mit Wasserstoff durchführt, der einen Kohlenmonoxidgehalt im Bereich von 50 bis 3000 ppm, insbesondere im Bereich von 100 bis 2000 ppm, speziell im Bereich von 200 bis 1000 ppm und ganz speziell im Bereich von 400 bis 800 ppm aufweist.

Das Hydrierprodukt lässt sich durch dem Fachmann an sich bekannte Verfahren, wie z. B. durch Destillation und/oder Entspannungsverdampfung, aus dem Hydriergemisch abtrennen und der zurückbleibende Katalysator im Rahmen weiterer Umsetzungen nutzen. Im Rahmen der bevorzugten Ausführungsform verzichtet man vorteilhaft auf den Zusatz von Lösemitteln und führt die genannten Umsetzungen im umzusetzenden Substrat bzw. dem Produkt und gegebenenfalls in hochsiedenden Nebenprodukten als Lösemedium durch. Insbesondere bevorzugt ist die kontinuierliche Reaktionsführung unter Wiederverwendung bzw. Rückführung des erfindungsgemäß stabilisierten homogenen Katalysators.

In einer bevorzugten Ausführungsform wird eine prochirale α,β-ungesättigte Carbonylverbindung hydriert. Eine prochirale α,β-ungesättigteCarbonylverbindung kann durch Additionsreaktion an die olefinische Doppelbindung ein Chiralitätszentrum ausbilden. Hierzu trägt die Doppelbindung vier verschiedene Substituenten. Die prochirale α,β-ungesättigte Carbonylverbindung ist vorzugsweise ausgewählt unter Verbindungen der allgemeinen Formel (V) 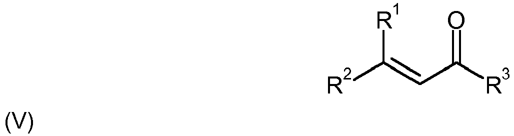 worin
- R¹, R²: voneinander verschieden sind und jeweils für einen unverzweigten, verzweigten oder cyclischen Kohlenwasserstoffrest mit 1 bis 25 Kohlenstoffatomen stehen, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen,
- R³: für Wasserstoff oder für einen unverzweigten, verzweigten oder cyclischen Kohlenwasserstoffrest mit 1 bis 25 Kohlenstoffatomen steht, der gesättigt ist oder eine oder mehrere, nicht konjugierte ethylenische Doppelbindungen aufweist, und der unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen, oder
- R³: gemeinsam mit einem der Reste R¹ oder R² auch eine 3 bis 25-gliedrige Alkylengruppe bedeuten kann worin 1, 2, 3 oder 4 nicht benachbarte CH₂-Gruppen durch O oder N-R^{5c} ersetzt sein können, wobei die Alkylengruppe gesättigt ist oder eine oder mehrere, nicht konjugierte ethylenische Doppelbindungen aufweist, und wobei die Alkylengruppe unsubstituiert ist oder einen oder mehrere gleiche oder verschiedene Substituenten trägt, die ausgewählt sind unter OR⁴, NR^{5a}R^{5b}, Halogen, C₁-C₄-Alkyl, C₆-C₁₀-Aryl und Hetaryl mit 5 bis 10 Ringatomen, wobei zwei Substituenten auch gemeinsam für eine 2 bis 10-gliedrige Alkylengruppe stehen können, wobei die 2- bis 10-gliedrige Alkylengruppe gesättigt ist oder eine oder mehrere, nicht konjugierte ethylenische Doppelbindungen aufweist, und wobei die 2- bis 10-gliedrige Alkylengruppe unsubstituiert ist oder einen oder mehrere gleiche oder verschiedene Substituenten trägt, die ausgewählt sind unter OR⁴, NR^{5a}R^{5b}, Halogen, C₆-C₁₀-Aryl und Hetaryl mit 5 bis 10 Ringatomen;
wobei
- R⁴: für Wasserstoff, C₁-C₆-Alkyl, C₆-C₁₀-Aryl, C₆-C₁₄-Aryl-C₁-C₁₀-alkyl, oder C₁-C₁₀-Alkyl-C₆-C₁₄-aryl- steht;
- R^{5a}, R^{5b}: jeweils unabhängig voneinander Wasserstoff, C₁-C₆-Alkyl, C₆-C₁₀-Aryl, C₆-C₁₄-Aryl-C₁₀-C₁₀-alkyl oder C₁-C₁₀-Alkyl-C₆-C₁₄-aryl bedeuten oder
- R^{5a} und R^{5b}: gemeinsam auch eine Alkylenkette mit 2 bis 5 Kohlenstoffatomen, die durch N oder O unterbrochen sein kann, bedeuten können; und
- R^{5c}: für Wasserstoff, C₁-C₆-Alkyl, C₆-C₁₀-Aryl, C₆-C₁₄-Aryl-C₁-C₁₀-alkyl oder C₁-C₁₀-Alkyl-C₆-C₁₄-ary- steht.

In bevorzugten Ausführungsformen ist die prochirale α,β-ungesättigte Carbonylverbindung ausgewählt unter Verbindungen der allgemeinen Formeln (Va) und (Vb) 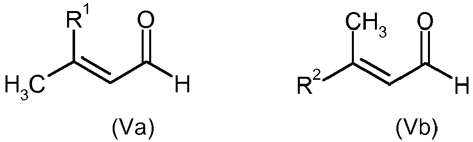 worin
- R¹, R²: jeweils für einen unverzweigten oder verzweigten Kohlenwasserstoffrest mit 2 bis 25 Kohlenstoffatomen steht, der gesättigt ist oder 1, 2, 3, 4 oder 5 nicht konjugierte ethylenische Doppelbindungen aufweist.

Eine besonders bevorzugte Ausführungsform betrifft ein Verfahren zur Herstellung von optisch aktivem Citronellal der Formel (VI) 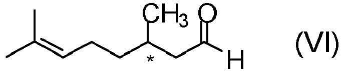
worin * das Asymmetriezentrum bezeichnet;
durch asymmetrische Hydrierung von Geranial der Formel (Va-1) oder von Neral der Formel (Vb-1) 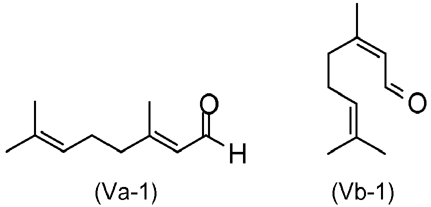
oder einer Neral und Geranial enthaltenden Mischung. Eine Neral und Geranial enthaltende Mischung ist unter der Bezeichnung Citral bekannt.

Man kann das so erhältliche optisch aktive Citronellal der Formel (VI) einer Cyclisierung zu optisch aktivem Isopulegol unterziehen und das optisch aktive Isopulegol zu optisch aktivem Menthol hydrieren.

Die Erfindung wird durch die beigefügte Figur näher veranschaulicht.

### Figur 1

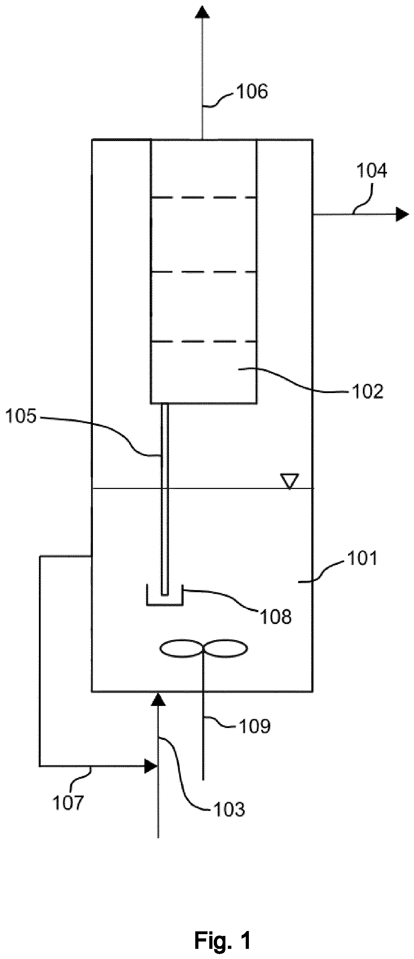

Fig. 1 zeigt schematisch einen erfindungsgemäßen Reaktor.

Der Reaktor umfasst eine rückvermischte Zone 101 und eine Zone begrenzter Rückvermischung 102, deren Rückvermischung durch eingebaute Böden begrenzt ist. Über eine Injektionsdüse (nicht dargestellt) werden ein Gas und eine Flüssigkeit aus der Leitung 103 der rückvermischten Zone 101 zugeführt.

Die rückvermischte Zone 101 weist einen Gasauslass 104 auf, über den nicht umgesetztes Gas ausgeführt wird. Über das Steigrohr 105, dessen unteres Ende unterhalb des Flüssigkeitsstandes angeordnet ist, steigt Flüssigkeit von der rückvermischten Zone 101 in die Zone begrenzter Rückvermischung 102 auf. Über den Reaktionsproduktauslass 106 wird Reaktionsprodukt ausgeführt.

Die rückvermischte Zone ist als Schlaufenreaktor mit externem Kreislauf 107 gestaltet. So befindet sich im unteren Bereich der rückvermischten Zone ein Abzug, über den das Reaktionsgemisch über den externen Kreislauf 107 mittels einer Pumpe (nicht dargestellt) in die Leitung 103 und somit der rückvermischten Zone 101 wieder zugeführt wird.

Der Reaktor umfasst einen Umlenkwehr 108, welcher den Eintritt von Gas in das Steigrohr 105 im Wesentlichen verhindert.

Anstelle des externen Kreislaufs 107 kann die rückvermischte Zone 101 einen Rührer 109 aufweisen.

## Patentansprüche

1. Reaktor zur Durchführung einer gas/flüssig-zweiphasigen Hochdruckreaktion mit einem schäumenden Medium, mit
einem von einem zylindrischen, vertikal ausgerichteten länglichen Mantel, einem Boden und einer Haube gebildeten Innenraum,
wobei der Innenraum durch Einbauten in eine rückvermischte Zone (101) und eine Zone begrenzter Rückvermischung (102) unterteilt ist, wobei die rückvermischte Zone (101) und die Zone begrenzter Rückvermischung (102) vom Reaktionsgemisch nacheinander durchströmbar sind, wobei die rückvermischte Zone Mittel zum Einleiten von Gas und Flüssigkeit und einen Gasauslass (104) aufweist sowie mindestens ein Mischorgan (109) aufweist, welches ausgewählt ist unter einem Rührer, einer Strahldüse und Mitteln zum Einpressen des Gases, und die Zone begrenzter Rückvermischung (102) einen Reaktionsproduktauslass (106) aufweist,
einem ersten zylindrischen Einbauelement, welches sich im Innenraum in Längsrichtung des Reaktors erstreckt und welches die Zone begrenzter Rückvermischung (102) von der rückvermischten Zone (101) abgrenzt,
rückvermischungshindernden zweiten Einbauelementen in Form von Füllkörpern, strukturierten Packungen oder flüssigkeitsdurchlässigen Böden, welche in der Zone begrenzter Rückvermischung (102) angeordnet sind,
einem Steigrohr (105), dessen unteres Ende innerhalb der rückvermischten Zone (101) angeordnet ist und dessen oberes Ende in die Zone begrenzter Rückvermischung (102) mündet, so dass durch das Steigrohr (105) Flüssigkeit von der rückvermischten Zone (101) in die Zone begrenzter Rückvermischung (102) aufsteigen kann, und
einem am unteren Ende des Steigrohrs (105) angeordneten Einbauelement (108), das den Eintritt von Gas in das Steigrohr (105) verhindert, welches Einbauelement (108) ausgewählt ist unter einem Umlenkwehr und einem U-Rohr,
wobei die Zone begrenzter Rückvermischung (102) von unten angeströmt wird.

2. Reaktor nach Anspruch 1, wobei das Volumenverhältnis von rückvermischter Zone (101) zu Zone begrenzter Rückvermischung (102) im Bereich von 0,25:1 bis 4:1 liegt.

3. Reaktor nach Anspruch 1 oder 2, umfassend mindestens ein drittes Einbauelement, welches in der oberen Hälfte der rückvermischten Zone (101) angeordnet ist und eine Oberfläche aufweist, welche die Koaleszenzneigung schäumender Medien unterstützt.

4. Reaktor nach einem der vorhergehenden Ansprüche, wobei das erste Einbauelement konzentrisch zum Mantel angeordnet ist, so dass die Zone begrenzter Rückvermischung (102) einen kreisförmigen horizontalen Querschnitt aufweist.

5. Verfahren zur Durchführung einer kontinuierlichen gas/flüssig-zweiphasigen Hochdruckreaktion, bei dem man in einen Reaktor nach einem der vorhergehenden Ansprüche ein Gas und eine Flüssigkeit in die rückvermischte Zone (101) einführt, Flüssigkeit durch das Steigrohr (105) von der rückvermischten Zone (101) in die Zone begrenzter Rückvermischung (102) aufsteigen lässt, nicht umgesetztes Gas über den Gasauslass (104) zumindest teilweise ausführt, und ein Reaktionsprodukt am Reaktionsproduktauslass (106) abzieht.

6. Verfahren nach Anspruch 5 zur Präformierung eines homogenen Rhodium-Hydrierkatalysators, der mindestens einen CO-Liganden enthält, wobei die Flüssigkeit einen gelösten CO-defizienten Rhodium-Hydrierkatalysator umfasst und wobei das Gas Wasserstoff und Kohlenmonoxid enthält, wobei aus der Reaktion des CO-defizienten Rhodium-Hydrierkatalysators mit dem Gas ein hydrieraktiver Rhodium-Hydrierkatalysator erhalten wird.

7. Verfahren nach Anspruch 6, wobei der CO-defiziente Rhodium-Hydrierkatalysator wenigstens einen chiralen Liganden, insbesondere Chiraphos, aufweist.

8. Verfahren nach Anspruch 6 oder 7, wobei die Flüssigkeit eine Verbindung der Formel (IV) umfasst, 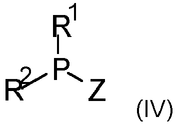 worin Z in Formel (IV) für eine Gruppe CHR³R⁴ steht und worin die Variablen R¹, R², R³, R⁴ unabhängig voneinander und insbesondere gemeinsam die folgenden Bedeutungen aufweisen:
R¹, R²: sind gleich oder verschieden und stehen für Phenyl, das unsubstituiert ist oder 1, 2 oder 3 Substituenten trägt, die ausgewählt sind unter Methyl und Methoxy, wobei R¹ und R² jeweils insbesondere für unsubstituiertes Phenyl stehen;
R³ steht für C₁- bis C₄-Alkyl, insbesondere für Methyl;
R⁴ steht für C₁- bis C₄-Alkyl, das eine Gruppe P(=O)R^{4a}R^{4b} trägt und insbesondere eine Gruppe CH₂-P(=O)R^{4a}R^{4b} oder CH(CH₃)-P(=O)R^{4a}R^{4b} bedeutet;
wobei
R^{4a}, R^{4b}: gleich oder verschieden sind und für Phenyl stehen, das unsubstituiert ist oder 1, 2 oder 3 Substituenten trägt, die ausgewählt sind unter Methyl und Methoxy, wobei besonders bevorzugt R^{4a} und R^{4b} jeweils für unsubstituiertes Phenyl stehen.

9. Verfahren nach einem der Ansprüche 6 bis 8, wobei man das Reaktionsprodukt der Präformierung zusammen mit einem zu hydrierenden Substrat einer asymmetrischen Hydrierungsreaktion zuführt, wobei ein Hydrierreaktionsgemisch erhalten wird, man vom Hydrierreaktionsgemisch ein Hydrierprodukt abtrennt und einen Rückstand mit CO-defizientem Rhodium-Hydrierkatalysator erhält, der der Präformierung zuführt wird.

10. Verfahren zur Herstellung von optisch aktivem Menthol, bei dem man optisch aktives Citronellal der Formel (VI) 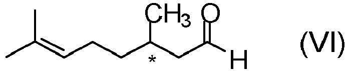
worin * das Asymmetriezentrum bezeichnet;
nach einem Verfahren des Anspruchs 9 herstellt, wobei die Hydrierungsreaktion die asymmetrische Hydrierung von Geranial der Formel (Va-1) oder von Neral der Formel (Vb-1) 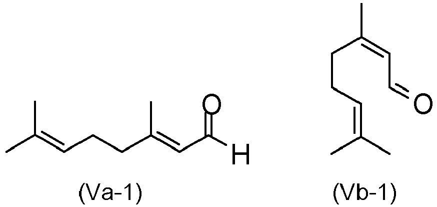
oder einer Neral und Geranial enthaltenden Mischung umfasst,
das optisch aktive Citronellal der Formel (VI) einer Cyclisierung zu optisch aktivem Isopulegol unterzieht und das optisch aktive Isopulegol zu optisch aktivem Menthol hydriert.

## Claims

1. A reactor for performing a gas/liquid biphasic high-pressure reaction with a foaming medium, comprising an interior formed by a cylindrical, vertically oriented elongate shell, a bottom and a cap,
wherein the interior is divided by internals into a backmixed zone (101) and a zone of limited backmixing (102), wherein the backmixed zone (101) and the zone of limited backmixing (102) are consecutively traversable by the reaction mixture, wherein the backmixed zone comprises means for introducing gas and liquid and a gas outlet (104) and also comprises at least one mixing apparatus (109) selected from a stirrer, a jet nozzle and means for injecting the gas, and the zone of limited backmixing (102) comprises a reaction product outlet (106),
a first cylindrical internal element which in the interior extends in the longitudinal direction of the reactor and which delimits the zone of limited backmixing (102) from the backmixed zone (101), backmixing-preventing second internal elements in the form of random packings, structured packings or liquid-permeable trays arranged in the zone of limited backmixing (102),
a riser tube (105) whose lower end is arranged within the backmixed zone (101) and whose upper end opens into the zone of limited backmixing (102) so that liquid from the backmixed zone (101) can ascend into the zone of limited backmixing (102) through the riser tube (105), and
an internal element (108) which is arranged at the lower end of the riser tube (105) and which prevents entry of gas into the riser tube (105), which internal element (108) is selected from a deflection weir and a U-tube,
wherein flow into the zone of limited backmixing (102) enters from below.

2. The reactor according to claim 1, wherein the volume ratio of the backmixed zone (101) to the zone of limited backmixing (102) is in the range from 0.25:1 to 4:1.

3. The reactor according to claim 1 or 2, comprising at least one third internal element which is arranged in the upper half of the backmixed zone (101) and has a surface which promotes the propensity to coalescence of foaming media.

4. The reactor according to any of the preceding claims, wherein the first internal element is arranged concentrically to the shell so that the zone of limited backmixing (102) has a circular horizontal cross section.

5. A process for performing a continuous gas/liquid biphasic high-pressure reaction in which in a reactor according to any of the preceding claims a gas and a liquid are introduced into the backmixed zone (101), liquid is allowed to ascend from the backmixed zone (101) into the zone of limited backmixing (102) through the riser tube (105), unconverted gas is at least partially discharged via the gas outlet (104) and a reaction product is withdrawn at the reaction product outlet (106).

6. The process according to claim 5 for preforming a homogeneous rhodium hydrogenation catalyst comprising at least one CO ligand, wherein the liquid comprises a dissolved CO-deficient rhodium hydrogenation catalyst and wherein the gas comprises hydrogen and carbon monoxide, wherein the reaction of the CO-deficient rhodium hydrogenation catalyst with the gas affords a hydrogenation-active rhodium hydrogenation catalyst.

7. The process according to claim 6, wherein the CO-deficient rhodium hydrogenation catalyst comprises at least one chiral ligand, in particular chiraphos.

8. The process according to claim 6 or 7, wherein the liquid comprises a compound of formula (IV) 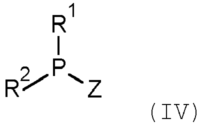 wherein Z in formula (IV) represents a CHR³R⁴ group and wherein the variables R¹, R², R³, R⁴ independently of one another and especially jointly are as follows:
R¹, R²: are identical or different and represent phenyl which is unsubstituted or bears 1, 2 or 3 substituents selected from methyl and methoxy, wherein R¹ and R² each especially represent unsubstituted phenyl;
R³ represents C₁- to C₄-alkyl, especially methyl;
R⁴ represents C₁- to C₄-alkyl bearing a P(=O)R^{4a}R^{4b} group and especially a CH₂-P(=O) R^{4a}R^{4b} or CH(CH₃)-P (=O) R^{4a}R^{4b} group;
wherein
R^{4a}, R^{4b}: are identical or different and represent phenyl which is unsubstituted or bears 1, 2 or 3 substituents selected from methyl and methoxy, wherein R^{4a} and R^{4b} each particularly preferably represent unsubstituted phenyl.

9. The process according to any of claims 6 to 8, wherein the reaction product of the preforming is supplied to an asymmetric hydrogenation reaction together with a substrate to be hydrogenated to afford a hydrogenation reaction mixture and a hydrogenation product is separated from the hydrogenation reaction mixture to afford a residue comprising CO-deficient rhodium hydrogenation catalyst which is supplied to the preforming.

10. A process for producing optically active menthol in which optically active citronellal of formula (VI) 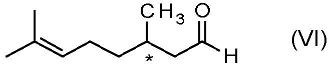
wherein * denotes the asymmetric center;
is produced by a process according to claim 9, wherein the hydrogenation reaction comprises the asymmetric hydrogenation of geranial of formula (Va-1) or of neral of formula (Vb-1) 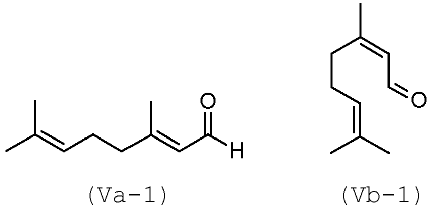
or a mixture comprising neral and geranial,
the optically active citronellal of formula (VI) is subjected to a cyclization to afford optically active isopulegol and the optically active isopulegol is hydrogenated to afford optically active menthol.

## Revendications

1. Réacteur destiné à réaliser une réaction à haute pression diphasique gaz/liquide avec un milieu moussant, ledit réacteur comprenant
un espace intérieur formé par une enveloppe allongée cylindrique orientée verticalement, un fond et un capot, l'espace intérieur étant divisé par des chicanes en une zone de remélange (101) et une zone de remélange limité (102), la zone de remélange (101) et la zone de remélange limité (102) pouvant être traversées successivement par le mélange réactionnel, la zone de remélange comportant des moyens d'introduction de gaz et de liquide et une sortie de gaz (104) et comportant également au moins un organe mélangeur (109) qui est choisi parmi un agitateur, une buse et des moyens d'injection du gaz, et la zone de remélange limité (102) comportant une sortie de produit de réaction (106),
un premier élément intégré cylindrique qui s'étend dans l'espace intérieur dans la direction longitudinale du réacteur et qui forme une délimitation entre la zone de remélange limité (102) et la zone de remélange (101),
des deuxièmes éléments intégrés, empêchant le remélange, qui se présentent sous la forme de corps de remplissage, de garnissages structurés ou de fonds perméables aux liquides et qui sont disposés dans la zone de remélange limité (102),
une colonne montante (105) dont l'extrémité inférieure est disposée à l'intérieur de la zone de remélange (101) et dont l'extrémité supérieure débouche dans la zone de remélange limité (102) de sorte que le liquide puisse monter à travers la colonne montante (105) de la zone de remélange (101) jusque dans la zone de remélange limité (102), et
un élément intégré (108) qui est disposé à l'extrémité inférieure de la colonne montante (105) et qui empêche l'entrée de gaz dans la colonne montante (105), lequel élément intégré (108) est choisi parmi un élément antidéflecteur et un tube en U,
la zone de remélange limité (102) recevant un écoulement depuis le bas.

2. Réacteur selon la revendication 1, le rapport volumique de la zone de remélange (101) à la zone de remélange limité (102) étant dans la gamme allant de 0,25:1 à 4:1.

3. Réacteur selon la revendication 1 ou 2, comprenant au moins un troisième élément intégré qui est disposé dans la moitié supérieure de la zone de remélange (101) et une surface qui favorise la tendance des milieux moussants à coalescer.

4. Réacteur selon l'une des revendications précédentes, le premier élément intégré étant disposé concentriquement à l'enveloppe de sorte que la zone de remélange limité (102) présente une section transversale horizontale circulaire.

5. Procédé de réalisation d'une réaction à haute pression diphasique gaz/liquide continue, procédé dans lequel on introduit dans un réacteur selon l'une des revendications précédentes un gaz et un liquide dans la zone de remélange (101), on fait monter le liquide à travers la colonne montante (105) de la zone de remélange (101) jusque dans la zone de remélange limité (102), on évacue au moins partiellement le gaz n'ayant pas réagi par la sortie de gaz (104) et on soutire un produit de réaction à la sortie de produit de réaction (106) .

6. Procédé selon la revendication 5 destiné à préformer un catalyseur d'hydrogénation de rhodium homogène qui contient au moins un ligand CO, le liquide comprenant un catalyseur d'hydrogénation de rhodium déficient en CO dissous et le gaz contenant de l'hydrogène et du monoxyde de carbone, un catalyseur d'hydrogénation de rhodium à hydrogénation active étant obtenu à partir de la réaction du catalyseur d'hydrogénation de rhodium déficient en CO avec le gaz.

7. Procédé selon la revendication 6, le catalyseur d'hydrogénation de rhodium déficient en CO comportant au moins un ligand chiral, en particulier du chiraphos.

8. Procédé selon la revendication 6 ou 7, le liquide comprenant un composé de formule (IV) 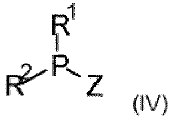 où Z dans la formule (IV) est un groupement CHR³R⁴ et les variables R¹, R², R³, R⁴ ont indépendamment et notamment conjointement les significations suivantes :
R¹, R² sont identiques ou différents et représentent un phényle non substitué ou portant 1, 2 ou 3 substituants choisis parmi un méthyle et un méthoxy, R¹ et R² représentant chacun notamment un phényle non substitué ;
R³ représente un alkyle en C₁ à C₄, en particulier un méthyle ;
R⁴ est un alkyle en C₁ à C₄ portant un groupe P (=O) R^{4a}R^{4b} et en particulier un groupe CH₂-P(=O)R^{4a}R^{4b} ou CH(CH₃)-P(=O)R^{4a}R^{4b} ;
R^{4a}, R^{4b} étant identiques ou différents et représentant un phényle non substitué ou portant 1, 2 ou 3 substituants choisis parmi un méthyle et un méthoxy, R^{4a} et R^{4b} représentant chacun de manière particulièrement préférée un phényle non substitué.

9. Procédé selon l'une des revendications 6 à 8, le produit de réaction du préformage étant amené conjointement avec un substrat à hydrogéner à une réaction d'hydrogénation asymétrique, un mélange de réaction d'hydrogénation étant obtenu, un produit d'hydrogénation étant séparé du mélange de réaction d'hydrogénation et un résidu contenant un catalyseur d'hydrogénation de rhodium déficient en CO étant obtenu qui est amené au préformage.

10. Procédé de production de menthol optiquement actif, procédé dans lequel du citronellal optiquement actif de formule (VI) 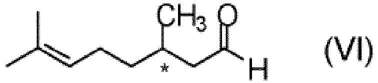
où * désigne le centre d'asymétrie,
est produit selon un procédé de la revendication 9, la réaction d'hydrogénation comprenant l'hydrogénation asymétrique de géranial de formule (Va-1) ou de néral de formule (Vb-1) 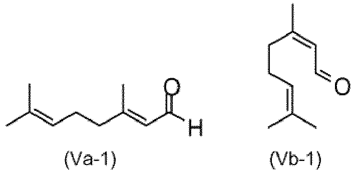
ou d'un mélange contenant du néral et du géranial,
le citronellal optiquement actif de formule (VI) est soumis à une cyclisation pour former de l'isopulégol optiquement actif et l'isopulégol optiquement actif est hydrogéné pour former du menthol optiquement actif.
